# EUROPÄISCHE PATENTSCHRIFT

(11) **EP 1 459 713 B1**
(45) Veröffentlichungstag und Bekanntmachung des Hinweises auf die Patenterteilung: **01.03.2006**
(21) Anmeldenummer: 04006092.3
(22) Anmeldetag: 15.03.2004
(51) Int. Cl.: A61F 5/01, A61F 13/06

(54) **Schlauchförmige Kompressionsbandage**
Tubular compression bandage
Bandage tubulaire de compression

(30) Priorität: 21.03.2003 DE 10312656
(43) Veröffentlichungstag der Anmeldung: 22.09.2004
(73) Patentinhaber: Bauerfeind AG, 07937 Zeulenroda (DE)
(72) Erfinder: Hess, Heinrich, Prof., 66271 Kleinblittersdorf (DE)
(74) Vertreter: Bardehle, Heinz

(56) Entgegenhaltungen:
- US-A- 3 831 467
- US-A- 5 016 621
- US-A- 5 254 122
- US-A- 6 080 121
- US-B1- 6 425 876

## Beschreibung

Die Erfindung bezieht sich auf eine schlauchförmige Kompressionsbandage aus elastischem Textilmaterial, die mit mehreren, die Bandage benachbart umfassenden Spannbändern versehen ist. Dokument US-A-6 425 876 stellt der nächste Stand der Technik dar.

Eine derartige Bandage ist z.B. in der deutschen Patentschrift 44 19 260 offenbart. Die an dieser Bandage angebrachten Spannbänder erstrecken sich jeweils in Umfangsrichtung über eine kurze Länge der Bandage und dienen dazu, den von ihnen überbrückten Bereich mehr oder minder stark zusammenzuziehen. Mit dieser Bandage lässt sich beim Anziehen der Spannbänder ein von der Bandage radial nach innen ausgehender Druck auf das betreffende Körperteil ausüben, wobei aufgrund der in Umfangsrichtung der Bandage verlaufenden Richtung der Spannbänder die Bandage allein diese Druckwirkung ausüben kann.

Bandagen mit mehr oder minder weit umlaufenden Spannbändern gibt es im Stand der Technik in vielfältiger Ausführung. Hierzu sei beispielsweise auf die PCT-Schrift WO 01/03624 verwiesen, bei der es sich gemäß Figur 2 um eine längsgeschlitzte Bandage mit in ausschließlicher Umfangsrichtung praktisch vollständig umlaufenden Spannbändern handelt. In der US-PS 3,856,008 ist ebenfalls eine mit einem Längsschlitz versehene Bandage offenbart, bei der der Längsschlitz durch in Umfangsrichtung verlaufende Spannbänder überbrückt wird, um durch wahlweise Anlegung der Bänder einen unterschiedlichen, in Radialrichtung verlaufenden Druck auf das betreffende Körperteil auszuüben. In der US-PS 4,366,813 ist eine schlauchförmige Bandage für das Kniegelenk offenbart, bei der zwecks Konzentration der Wirkung der Bandage auf das Kniegelenk sowohl oberhalb des Kniegelenks als auch unterhalb des Kniegelenks etwa unter 45° verlaufende Spannbänder angebracht sind, die derart gegenläufig gerichtet sind, dass sich die von den schrägverlaufenden Spannbändern ausgeübten Kräfte gegenseitig kompensieren, wodurch die Bandage gegen Verschieben gesichert und an ihrem Sitz im Bereich des Kniegelenks festgehalten wird.

Kompressionsbandagen werden insbesondere dann verwendet, wenn eine Verletzung von Muskelfasern, insbesondere also ein Muskelriss, vorliegt. Bei der Behandlung derartiger Verletzungen hat sich gezeigt, dass eine insbesondere zur Vermeidung von Blutergüssen angelegte Bandage, die nur radial nach innen gerichteten Druck ausübt, nicht zu optimalen Ergebnissen führt.

Der Erfindung liegt daher die Aufgabe zugrunde, eine schlauchförmige Kompressionsbandage der eingangs angegebenen Gestaltung zu schaffen, bei der die therapeutische Wirkung gegenüber den bekannten Bandagen wesentlich verbessert ist. ErfindungsgemäB geschieht dies dadurch, dass die Spannbänder mit ihren Anfängen an einem mit der Außenfläche der Bandage verbundenen Anfangslängsstreifen befestigt sind und umlaufend paarweise in Schrägführung von bis zu 30° zur Längsrichtung der Bandage zu ihrem Ende auf der dem Anfangslängsstreifen gegenüberliegenden Seite zu fischgrätenartig angeordneten Paaren von Spannösen geführt und in diesen zu Klettverschlüssen an der Bandage zurückgelenkt sind, wobei der Grad der Schrägführung durch wahlweise Festlegung des Anfangs bzw. Endes der Spannbänder in Längsrichtung der Bandage einstellbar ist.

Bei der erfindungsgemäßen Bandage werden ein nach innen gerichteter Druck als auch ein in Längsrichtung des betreffenden Körperteils verlaufender Druck miteinander kombiniert, da nämlich die Spannbänder, die auf jeden Fall einen nach innen gerichteten Druck ausüben, aufgrund ihrer Schrägführung zusätzlich in dem behandelten Körperteil einen in dessen Längsrichtung verlaufenden Druck hervorrufen, der im Sinne der betreffenden Richtung der Muskelfasern dafür sorgt, dass die Muskelfasern zusammengezogen werden. Mit dem Anliegen dieser Bandage in einem Bereich, der wesentlich auch neben der eigentlichen Verletzungsstelle liegt, wird von der Bandage ein Zug auf das betreffende Körperteil ausgeübt, der in Richtung der Muskelfasern verläuft und dafür sorgt, dass der verletzte Bereich die Tendenz erhält, zusammengeschoben zu werden, womit einem entstehenden Blutgerinnsel der dafür notwendige Platz genommen und die entstehende Narbe möglichst klein gehalten wird, um bei der Muskelfunktion möglichst wenig zu stören.

Die Stärke des auf das betreffende Körperteil ausgeübten, in dessen Längsrichtung verlaufenden Zuges hängt von dem Grad der Schrägführung der Spannbänder ab, die sinnvoller Weise bis zu 30° zur Längsrichtung der Bandage zu führen sind. Durch die wahlweise Festlegung des Anfangs bzw. Endes der Spannbänder in Längsrichtung der Bandage lässt sich diese an das betreffende Körperteil unmittelbar beim Anlegen der Bandage so anpassen, wie dies die Gestaltung des betreffenden Körperteils erfordert, wobei also die Dicke des Körperteils und ggf. dessen Konizität direkt berücksichtigt werden kann und damit auch der notwendige Zug auf das betreffende Körperteil hinsichtlich seiner Kraft wahlweise einstellbar ist.

Für die wahlweise Festlegung des Anfangs der Spannbänder kann man die Paare von Spannösen als Reihe in Längsrichtung der Bandage an einem an der Bandage befestigten Endlängsstreifen in wahlweise Lage bezüglich der Längsrichtung der Bandage festlegen.

In entsprechender Weise kann man auch den Anfangslängsstreifen, an dem Spannbänder mit ihren Anfangsenden verbunden sind, als Klettverschlussstreifen ausbilden. Hierdurch ist es möglich, den Grad der Schrägführung der Spannbänder entsprechend zu variieren. Diese Festlegung kann man entweder nur hinsichtlich der Anfänge der Spannbänder oder hinsichtlich der Enden der Spannbänder oder hinsichtlich beider Teile der Spannbänder vornehmen.

Eine zusätzliche Behandlung eines verletzten Muskels mit der erfindungsgemäßen Kompressionsbandage lässt sich dadurch erzielen, dass diese mit einer Druckpelotte versehen wird, was sich vorteilhaft dadurch ermöglichen lässt, dass die Bandage auf ihrer Innenseite eine Klettverschlussschicht trägt, an der eine mit Klettverschlussteilen versehene Druckpelotte wahlweise anbringbar ist. Je nach Stelle der Verletzung, die von der Kompressionsbandage überdeckt wird, wird dann zusätzlich die Druckpelotte eingelegt und übt dann ergänzend zu den von der Bandage ausgeübten Zug- und Druckkräften einen zusätzlichen Druck auf die verletzte Stelle aus.

Die wahlweise Anbringung der Pelotte ermöglicht es, diese vor ihrem Einsatz in der Bandage mit einem kühlenden Medium, z.B. Eiswasser, zu versehen, die dieses Medium aufsaugt, was bekanntlich bei der Behandlung der hier in Rede stehenden Verletzungen ständig angewendet wird.

In den Figuren ist ein Ausführungsbeispiel der Erfindung dargestellt. Es zeigen:
- Figur 1: die Kompressionsbandage mit Ansicht der Paare der Spannösen und einer relativ starken Schrägführung der Spannbänder,
- Figur 2: ebenfalls eine Ansicht der Paare der Spannösen mit einer geringeren Schrägführung der Spannbänder;
- Figur 3: eine Ansicht der gleichen Bandage, und zwar der Rückseite der Bandage mit dem die Anfangsenden der Spannbänder haltenden Anfangslängsstreifen;
- Figur 4: einen Schnitt durch die Bandage, angelegt an einem Oberschenkel gemäß Linie I-I aus Figur 1;
- Figur 5: die Kompressionsbandage angelegt an einem Oberschenkel mit Sicht auf die Paare der Spannösen;

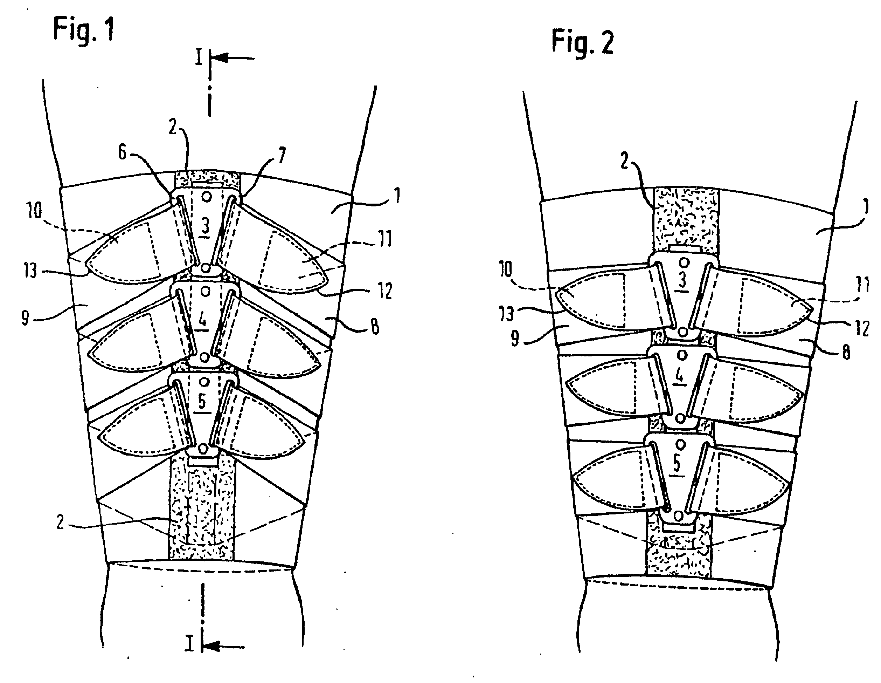

Die in der Figur 1 dargestellte schlauchförmige Kompressionsbandage besteht aus dem aus elastischem Textilmaterial gefertigten Schlauchstück 1, auf dessen Vorderseite außen der Anfangslängsstreifen 2 angebracht ist, der in Längsrichtung der Bandage verläuft. Auf dem Anfangslängsstreifen 2 sind drei Spannösenhalter 3, 4 und 5 befestigt, die jeweils ein Paar von Spannösen 6 und 7 aufweisen. Die Spannösen 6 und 7 sind, wie ersichtlich, gegenüber der Längsrichtung der Bandage schräg gestellt, was die Schrägführung der durch die Spannösen 6 und 7 hindurchgeführten Spannbänder 8 und 9 begünstigt. Die drei Spannösenhalter 3, 4 und 5 sind mittels Klettverschluss an dem Anfangslängsstreifen 2 befestigt, wozu dieser als Flauschstreifen ausgebildet ist und die Spannösenhalter 3, 4 und 5 mit entsprechenden Grip-Teilen versehen sind.

Die Spannbänder 8 und 9 sind durch die Spannösen 6 und 7 hindurchgezogen und danach zurückgelenkt, wobei sie ebenfalls mittels Klettverschluss 10 bzw. 11 an dem betreffenden Spannband 8 bzw. 9 befestigt sind, das zu diesem Zweck mit einer Flauschschicht versehen ist. In Figur 1 ist dargestellt, wie das Ende 12 des Spannbandes 8 zunächst noch nicht vollständig mit seinem Ende 12 zurückgelenkt ist.

Die Kompressionsbandage gemäß Figur 2 entspricht weitgehend derjenigen gemäß Figur 1, allerdings ist die Kompressionsbandage gemäß Figur 2 hinsichtlich der Schrägführung ihrer Spannbänder 8 und 9 anders eingestellt, nämlich durch eine entsprechende Festlegung der Spannösenhalter 3, 4 und 5 in einer etwas tieferen Lage derart, dass sich für die Spannbänder 8 und 9 eine gegenüber der Darstellung in Figur 1 geringere Schrägführung ergibt.

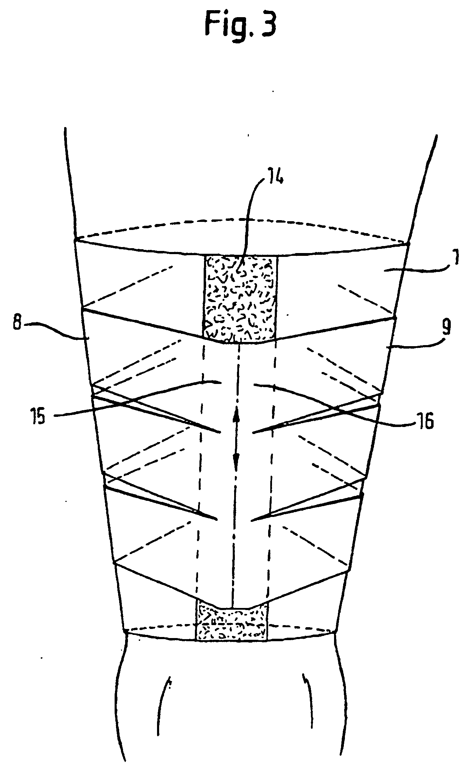

In Figur 3 ist die in den Figuren 1 bzw. 2 dargestellte Kompressionsbandage mit einer Sicht auf deren Rückseite dargestellt, nämlich auf den Anfangslängsstreifen 14, an dem die Anfänge 15 und 16 der Spannbänder 8 und 9 relativ weit unten an dem Anfangslängsstreifen 14 befestigt sind, und zwar mittels Klettverschluss, was dann zu einer entsprechenden sichtbaren Schräglage der Spannbänder 8 und 9 mit Rücksicht auf die auf der anderen Seite vorgenommene Befestigung der Spannösenhalter führt (siehe Figur 1 und 2).

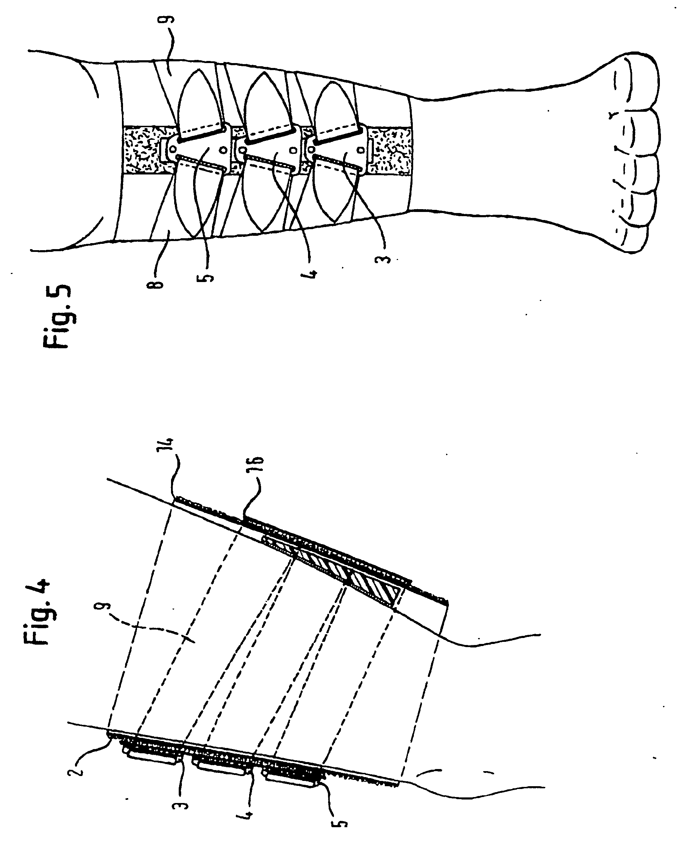

In der Figur 4 ist ein Schnitt längs der Linie I-I aus Figur 1 dargestellt, aus der die Lage der von dem betreffenden Körperteil verdeckten Spannbänder 9 ersichtlich ist (es handelt sich hier um einen Oberschenkel). Die Spannbänder 9 verlaufen von dem Anfangslängsstreifen 14 zu den Spannösenhaltern 3, 4 und 5, wo sie dann in der in den Figuren 1 und 2 dargestellten Weise zurückgelenkt werden. Die Spannösenhalter 3, 4 und 5 sind an dem Endlängsstreifen 2 festgelegt. Je nach Lage der Spannösenhalter 3, 4 und 5 und der Anfänge 16 an dem Anfangslängsstreifen 14 ergibt sich eine entsprechende Schräglage der Spannbänder 9, die anderen Anfänge 15 und Spannbänder 8 sind in der Schnittzeichnung gemäß Figur 4 nicht sichtbar. Weiterhin ist die Druckpelotte 17 dargestellt, die in die Bandage im Bereich des Anfangslängsstreifens 14 eingelegt ist und von einer innen an der Bandage angebrachten Klettverschlussschicht an der Einsatzstelle gehalten ist. Mit der Druckpelotte 17 wird örtlich je nach ihrer wahlweise gegebenen Lage ein besonderer Druck auf eine verletzte Stelle ausgeübt, womit jeweils eine besonders gewünschte Behandlung der Verletzung zusätzlich zu der Wirkung der Kompressionsbandage ermöglicht wird.

Zur Veranschaulichung von möglichen Anwendungsfällen der erfindungsgemäßen Kompressionsbandage ist in der Figur 5 ein Ausführungsbeispiel dargestellt. Dabei handelt es sich bei der Figur 5 in Sicht auf die Vorderseite um eine am Oberschenkel angelegte Bandage. Wie ersichtlich, verlaufen gemäß Figur 5 die Spannbänder 8 und 9 in einer Schrägführung, wie sie in den Figuren 1 bzw. 2 dargestellt ist, wobei also am Oberschenkel ein Zug der Spannbänder von unten hinten nach oben vom ausgeübt wird.

## Patentansprüche

1. Schlauchförmige Kompressionsbandage aus elastischem Textilmaterial, die mit mehreren, die Bandage (1) benachbart umfassenden Spannbändern (8, 9) versehen ist die mit ihren Anfängen (15, 16) an einem mit der Außenfläche der Bandage (1) verbundenen Anfangslängsstreifen (14) befestigt sind und umlaufend paarweise in Schrägführung zur Längsrichtung der Bandage (1) zu ihrem Ende auf der dem Anfangslängsstreifen (14) gegenüberliegenden Seite zu Klettverschlüssen (10, 11, 12, 13) an der Bandage (1) zurückgelenkt sind, wobei der Grad der Schrägführung durch wahlweise Festlegung des Anfangs bzw. Endes der Spannbänder (8, 9) in Längsrichtung der Bandage (1) einstellbar ist **dadurch gekennzeichnet, dass** die Spannbänder (8,9) paarweise in Schrägführung von bis zu 30° zur Längsrichtung der Bandage zu ihrem Ende zu fischgrätenartig angeordneten Paare von Spannösen geführt und in diesen zu den klettverschlüssen an der Bandage zurückgelegt sind.

2. Schlauchförmige Kompressionsbandage nach Anspruch 1, **dadurch gekennzeichnet, dass** die Paare von Spannösen (6, 7) als Reihe in Längsrichtung der Bandage (1) an einem an der Bandage (1) befestigten Endlängsstreifen (2) in wahlweiser Lage bezüglich der Längsrichtung der Bandage (1) festgelegt sind.

3. Schlauchförmige Kompressionsbandage nach Anspruch 2, **dadurch gekennzeichnet, dass** der Endlängsstreifen (2) als Klettverschlussstreifen ausgebildet ist.

4. Schlauchförmige Kompressionsbandage nach Anspruch 1, **dadurch gekennzeichnet, dass** der Anfangslängsstreifen (14) als Klettverschlussstreifen ausgebildet ist.

5. Schlauchförmige Kompressionsbandage nach einem der Ansprüche 1 bis 4, **dadurch gekennzeichnet, dass** die Bandage (1) auf ihrer Innenfläche eine Klettverschlussschicht trägt, an der eine mit Klettverschlussteilen versehene Druckpelottee (17) wahlweise anbringbar ist.

## Claims

1. Tubular compression bandage of elastic material provided with a plurality of tightening straps (8, 9) which encircle the bandage (1) adjacent to one another, are secured by means of their starting portions (15, 16) to a starting longitudinal strip (14) joined to the outer surface of the bandage (1) and are guided back circumferentially in pairs at an incline relative to the longitudinal direction of the bandage (1) by means of their terminating portions on the side opposite the starting longitudinal strip (14) to Velcro fasteners (10, 11, 12, 13) on the bandage (1), the degree of inclination being adjustable by fixing the starting or terminating portions of the tightening straps (8, 9) as required in the longitudinal direction of the bandage (1), **characterised in that** the tightening straps (8, 9) are guided in pairs at an angle of inclination of up to 30° relative to the longitudinal direction of the bandage by means of their terminating portions to pairs of tightening eyelets in a herring-bone arrangement and are folded back therein on to the Velcro fasteners on the bandage.

2. Tubular compression bandage according to claim 1, **characterised in that** the pairs of tightening eyelets (6, 7) are fixed in a row in the longitudinal direction of the bandage (1) to a terminating longitudinal strip (2) secured to the bandage (1), in the required position with respect to the longitudinal direction of the bandage (1).

3. Tubular compression bandage according to claim 2, **characterised in that** the terminating longitudinal strip (2) is designed as a Velcro fastening strip.

4. Tubular compression bandage according to claim 1, **characterised in that** the starting longitudinal strip (14) is designed as a Velcro fastening strip.

5. Tubular compression bandage according to one of claims 1 to 4, **characterised in that** the bandage (1) is provided on its inner surface with a Velcro fastening layer to which a pressure pad (17) provided with Velcro fastening portions can be attached as required.

## Revendications

1. Bandage de compression de forme tubulaire en matériau textile élastique pourvu de plusieurs bandes de serrage voisines (8, 9) qui enveloppent le bandage (1), qui sont fixées par leur début (15, 16) à une bande longitudinale de début (14) reliée à la surface extérieure du bandage (1) et qui, avec une orientation oblique par rapport à la direction longitudinale du bandage (1), sont ramenées par leurs fins et par paires sur le côté opposé à la bande longitudinale de début (14) jusqu'à des fermetures velcro (10, 11, 12, 13) placées sur le bandage (1), le degré d'oblicité pouvant être réglé en fixant de manière adéquate le début, respectivement la fin des bandes de serrage (8, 9) dans la direction longitudinale du bandage (1), **caractérisé en ce que** les bandes de serrage (8, 9) sont passées par leurs fins et par paires, avec une orientation oblique pouvant atteindre 30° par rapport à la direction longitudinale du bandage, dans des paires d'oeillets de tension disposées en arêtes de poisson, puis sont ramenées à travers celles-ci jusqu'aux fermetures velcro disposées sur le bandage.

2. Bandage de compression de forme tubulaire selon la revendication 1, **caractérisé en ce que** les paires d'oeillets de tension (6, 7) sont fixées sous la forme d'une rangée dans la direction longitudinale du bandage (1) sur une bande longitudinale de fin (2) fixée au bandage (1), dans une position modifiable à volonté dans la direction longitudinale du bandage (1).

3. Bandage de compression de forme tubulaire selon la revendication 2, **caractérisé en ce que** la bande longitudinale de fin (2) est conformée en bande de fermeture velcro.

4. Bandage de compression de forme tubulaire selon la revendication 1, **caractérisé en ce que** la bande longitudinale de début (14) est conformée en bande de fermeture velcro.

5. Bandage de compression de forme tubulaire selon l'une des revendications 1 à 4, **caractérisé en ce que** le bandage (1) porte, sur sa surface intérieure, une couche de fermeture, velcro sur laquelle peut être disposée sélectivement une pelote de compression (17) pourvue de portions de fermeture velcro.
